# EUROPÄISCHE PATENTSCHRIFT

(11) **EP 4 182 254 B1**
(45) Veröffentlichungstag und Bekanntmachung des Hinweises auf die Patenterteilung: **04.06.2025**
(21) Anmeldenummer: 21734109.8
(22) Anmeldetag: 17.06.2021
(51) Int. Cl.: B66B 31/02, B66B 25/00

(54) **PERSONENTRANSPORTANLAGE MIT DESINFEKTIONSEINRICHTUNG UND VERFAHREN ZU DEREN BETRIEB**
PASSENGER TRANSPORT SYSTEM WITH A DISINFECTING DEVICE AND METHOD FOR THE OPERATION OF THE SAME
SYSTÈME DE TRANSPORT DE PASSAGERS AVEC UN DISPOSITIF DE DÉSINFECTION ET PROCÉDÉ POUR LE FONCTIONNEMENT DU MÊME

(30) Priorität: 17.07.2020 EP 20186511
(43) Veröffentlichungstag der Anmeldung: 24.05.2023
(73) Patentinhaber: INVENTIO AG, 6052 Hergiswil (CH)
(72) Erfinder: HÄBERLE, Ulrich, 3002 Purkersdorf (AT); KLEEWEIN, Gerhard, 3021 Pressbaum (AT); WAGENLEITNER, Georg, 4575 Roßleithen (AT)
(74) Vertreter: Inventio AG
(86) Internationale Anmeldenummer: PCT/EP2021/066516
(87) Internationale Veröffentlichungsnummer: WO 2022/012855

(56) Entgegenhaltungen:
- WO-A2-2019/059858
- CN-A- 106 608 587
- CN-A- 111 346 247
- TW-A- 201 233 618

## Beschreibung

Die vorliegende Erfindung betrifft eine Personentransportanlage, insbesondere eine Personentransportanlage, welche über eine Desinfektionseinrichtung verfügt, sowie ein Verfahren zum Betreiben einer solchen Personentransportanlage.

Personentransportanlagen dienen dazu, Personen innerhalb von Gebäuden bzw.

Bauwerken zu befördern. Personentransportanlagen können beispielsweise als Fahrtreppen, Fahrsteige oder Aufzüge ausgestaltet sein.

An Oberflächen einer Personentransportanlage können sich Keime in Form von Viren, Bakterien und/oder Mikroben anlagern. Um deren Übertragung auf Passagiere der Personentransportanlage vermeiden zu können, kann in der Personentransportanlage eine Desinfektionseinrichtung vorgesehen sein. Die Desinfektionseinrichtung kann dazu eingerichtet sein, die betreffenden Oberflächen zu desinfizieren, beispielsweise indem dort befindliche Keime abgetötet und/oder entfernt werden.

Eine mögliche Ausgestaltung einer Desinfektionseinrichtung bedient sich Lichtquellen wie beispielsweise lichtemittierender Dioden (LEDs), welche eine keimtötende elektromagnetische Strahlung abgeben. Beispielsweise können hierfür sogenannte UVC-LEDs eingesetzt werden, welche hochenergetische Ultraviolett-Strahlung abgeben, welche auch als UVC-Strahlung bezeichnet wird und typischerweise einen Wellenlängenbereich von 100 - 300nm, meist 180 - 260nm, aufweist.

In der JP 2017 220328 A ist eine Vorrichtung zum Abschätzen einer Lebensdauer einer LED beschrieben. Die CN 106 608 587 A offenbart eine Personentransportanlage mit einer Desinfektionseinrichtung und im Handlauf integrierten Sensoren. Ein Steuergerät der Personentransportanlage ist über ein Kommunikationsmodul mit den Sensoren und der Desinfektionseinrichtung verbunden.

Es kann ein Bedarf an einer Personentransportanlage und einem Verfahren zu dessen Betrieb bestehen, welche es ermöglichen, die Personentransportanlage mit geringem Aufwand zuverlässig betreiben zu können und insbesondere Oberflächen an der Personentransportanlage zuverlässig und mit geringem Wartungs- und Kostenaufwand desinfizieren zu können.

Einem solchen Bedarf kann durch den Gegenstand gemäß einem der unabhängigen Ansprüche entsprochen werden. Vorteilhafte Ausführungsformen sind in den abhängigen Ansprüchen sowie der nachfolgenden Beschreibung definiert.

Gemäß einem ersten Aspekt der Erfindung wird eine Personentransportanlage vorgeschlagen, welche eine Personenfördereinrichtung zum Befördern von Personen innerhalb eines Bauwerks, ein Steuergerät zum Steuern eines Betriebs der Personenfördereinrichtung und eine Desinfektionseinrichtung zum Desinfizieren einer zu desinfizierenden Oberfläche an der Personenfördereinrichtung aufweist. Die Desinfektionseinrichtung weist eine Steuerung zum Steuern eines Betriebs der Desinfektionseinrichtung auf. Die Steuerung der Desinfektionseinrichtung ist über ein bidirektionales Kommunikationssystem an das Steuergerät der Personenfördereinrichtung angebunden. Das Steuergerät der Personentransportanlage und die Steuerung der Desinfektionseinrichtung sind dazu eingerichtet, dass Statussignale, welche Informationen über einen aktuellen Betrieb, einen aktuellen Zustand und/oder eine aktuelle Konfiguration der Desinfektionseinrichtung wiedergeben, von der Steuerung an das Steuergerät gesendet werden und das Steuergerät unter Berücksichtigung empfangener Statussignale Steuersignale generiert und an die Steuerung übermittelt, wobei die Steuerung den Betrieb der Desinfektionseinrichtung unter Berücksichtigung empfangener Steuersignale steuert. Des Weiteren ist die Steuerung der Desinfektionseinrichtung dazu eingerichtet, eine Desinfektionsintensität, mit der die Desinfektionseinrichtung die zu desinfizierende Oberfläche desinfiziert, abhängig von Steuersignalen zu steuern, welche der Steuerung von dem Steuergerät der Personentransportanlage übersendet werden. Diese Steuersignale geben hierbei einen mit einer aktuell zu bewirkenden Desinfektionsintensität korrelierenden Betriebsparameter der Desinfektionseinrichtung an.

Gemäß einem zweiten Aspekt der Erfindung wird ein Verfahren zum Betreiben einer Personentransportanlage gemäß einer Ausführungsform des ersten Aspekts beschrieben. Das Verfahren weist zumindest folgende Schritte auf:
- Übermitteln von Statussignalen, welche Informationen über einen aktuellen Betrieb, einen aktuellen Zustand und/oder eine aktuelle Konfiguration der Desinfektionseinrichtung wiedergeben, von der Steuerung an das Steuergerät, und
- Steuern eines Betriebs der Desinfektionseinrichtung unter Berücksichtigung empfangener Steuersignale, welche von dem Steuergerät unter Berücksichtigung empfangener Statussignale an die Steuerung übermittelt werden.

Die Statussignale können hierbei optional von dem Steuergerät an einen entfernt angeordneten Computer gesendet und dort verarbeitet werden. Ferner können optional Steuersignal von dem entfernt angeordneten Computer an das Steuergerät und von diesem weiter an die Steuerung übermittelt werden, um den Betrieb der Desinfektionseinrichtung zu steuern.

Mögliche Merkmale und Vorteile von Ausführungsformen der Erfindung können unter anderem und ohne die Erfindung einzuschränken als auf nachfolgend beschriebenen Ideen und Erkenntnissen beruhend angesehen werden.

Kurz zusammengefasst liegt eine wesentliche, der Erfindung zugrunde liegende Idee darin, dass erkannt wurde, dass eine Desinfektionseinrichtung in einer Personentransportanlage vorteilhafterweise nicht völlig unabhängig von anderen Komponenten der Personentransportanlage betrieben werden sollte. Einerseits kann die Art und Weise, wie die Desinfektionseinrichtung aktuell zu betreiben ist, von Bedingungen, die bei anderen Komponenten der Personentransportanlage vorherrschen, und/oder von Betriebsbedingungen der Personentransportanlage abhängen. Andererseits können auch auf die Desinfektionseinrichtung zutreffende Bedingungen und Zustände auf andere Komponenten der Personentransportanlage rückwirken. Daher wird vorgeschlagen, die Steuerung der Desinfektionseinrichtung und das Steuergerät der Personenfördereinrichtung mithilfe eines bidirektionalen Kommunikationssystems miteinander zu koppeln, so dass zwischen diesen Signale und Daten in beiden Richtungen übertragen werden können. Somit können einerseits Statussignale, welche Informationen betreffend die Desinfektionseinrichtung und insbesondere betreffend deren aktuellen Betrieb, aktuellen Zustand und/oder aktuelle Konfiguration angeben, von der Steuerung der Desinfektionseinrichtung über das bidirektionale Kommunikationssystem an das Steuergerät der Personenfördereinrichtung übermittelt werden, sodass diese Informationen beim Steuern der Personenfördereinrichtung berücksichtigt werden können und/oder diese Informationen gegebenenfalls von dem Steuergerät verarbeitet und/oder an andere Komponenten, insbesondere an einen externen Computer, zur dortigen Weiterverarbeitung übermittelt werden können. Andererseits können Steuersignale von dem Steuergerät der Personenfördereinrichtung generiert und an die Steuerung der Desinfektionseinrichtung übermittelt werden, sodass diese dann gemäß diesen Steuersignalen gesteuert werden kann. Dabei kann das Steuergerät beim Generieren der Steuersignale Statussignale, welche zuvor von der Steuerung der Desinfektionseinrichtung empfangen wurden, berücksichtigen. Letztendlich kann die Desinfektionseinrichtung daher beispielsweise in einer Weise, bei der sowohl die Informationen, die dem Steuergerät der Personenfördereinrichtung aufgrund von dessen Kommunikation mit anderen Komponenten der Personentransportanlage zur Verfügung stehen, als auch Informationen, die die Desinfektionseinrichtung selbst betreffen, gesteuert oder geregelt werden. Die Informationen können hierbei beispielsweise genutzt werden, um frühzeitig erkennen zu können, wenn sich Komponenten der Desinfektionseinrichtung ihrem Lebensdauerende nähern, sodass Wartungs- oder Ersetzungsmaßnahmen rechtzeitig geplant und eingeleitet werden können. Die Informationen können ferner beispielsweise auch genutzt werden, um Einflüsse des Betriebs der Desinfektionseinrichtung auf andere Komponenten der Personentransportanlage besser abschätzen zu können, beispielsweise indem die Statussignale betreffend die Desinfektionseinrichtung bei einer Pflege und Analyse eines digitalen Zwillings der Personentransportanlage berücksichtigt werden.

Nachfolgend werden mögliche Eigenschaften und Vorteile von Ausführungsformen der Personentransportanlage bzw. deren Betriebsverfahren detaillierter erläutert.

Die beschriebene Personentransportanlage kann als Fahrtreppe, Fahrsteig oder Aufzug ausgestaltet sein. Die Personenfördereinrichtung der Personentransportanlage ist mithilfe einer Kombination aus einer sich entlang eines Verfahrwegs verlagernden Einheit und einem Antrieb dazu ausgestaltet, eine oder vorzugsweise mehrere Personen innerhalb des Bauwerks zu befördern. Bei einer Ausgestaltung als Fahrtreppe weist die Personenfördereinrichtung hierzu ein Stufenband auf, bei dem mehrere Stufen in einer Verfahrrichtung hintereinander miteinander gekoppelt sind und welches von einem Antrieb umlaufend angetrieben werden kann. Ähnlich verfügt die Personenfördereinrichtung eines Fahrsteigs über ein Palettenband mit mehreren hintereinander angeordneten und miteinander gekoppelten Paletten, wobei das Palettenband ebenfalls von einem Antrieb umlaufend angetrieben werden kann. Bei einer Ausgestaltung als Aufzug weist die Personenfördereinrichtung zumindest eine Aufzugkabine auf, welche von einer Antriebseinrichtung innerhalb eines Aufzugschachts im Regelfall vertikal verlagert werden kann.

Das Steuergerät der Personentransportanlage ist dazu eingerichtet, den Betrieb der Personenfördereinrichtung und insbesondere deren Antrieb zu steuern. Hierbei können unter anderem eine Verfahraktivität, eine Verfahrrichtung und/oder eine Verfahrgeschwindigkeit gesteuert werden. Dabei können verschiedene Parameter und/oder Bedingungen berücksichtigt werden. Beispielsweise kann bei einer Fahrtreppe oder einem Fahrsteig mithilfe geeigneter Sensoren erkannt werden, ob sich eine oder mehrere Personen in einem Zugangsbereich und/oder auf dem Stufenband bzw. Palettenband befinden. Basierend auf Signalen solcher Sensoren kann ein Steuergerät dann den Antrieb der Fahrtreppe bzw. des Fahrsteigs ansteuern, um die Personen mithilfe der Personenfördereinrichtung in eine gewünschte Richtung und mit einer gewünschten Geschwindigkeit zu befördern. Bei einem Aufzug können z.B. Sensoren, Taster oder Ähnliches an Bedienpaneelen von Passagieren betätigt werden, um dem Steuergerät zu übermitteln, wohin die Aufzugkabine von dem Antrieb verfahren werden soll. Beim Steuern der Personenfördereinrichtung kann das Steuergerät ergänzend weitere Informationen berücksichtigen. Diese können von weiteren Sensoren stammen, welche beispielsweise Umgebungsbedingungen oder Belastungsbedingungen ermitteln. Die Informationen können auch aus anderen Quellen stammen. Beispielsweise können Informationen oder darin enthaltene Anweisungen von externen Geräten wie beispielsweise einem entfernt angeordneten Computer an das Steuergerät übermittelt werden, um dieses anzuweisen, wie die Personenfördereinrichtung aktuell betrieben bzw. angesteuert werden soll.

Die Desinfektionseinrichtung ist dazu eingerichtet, zumindest eine Oberfläche im Bereich der Personenfördereinrichtung zu desinfizieren. Insbesondere sollte die Desinfektionseinrichtung solche Oberflächen desinfizieren können, die häufig von Passagieren berührt werden und daher eine Verbreitung von Keimen zwischen verschiedenen Passagieren begünstigen können. Beispielsweise kann die Desinfektionseinrichtung eine Oberfläche eines Handlaufs einer Fahrtreppe oder eines Fahrsteigs desinfizieren. In einem Aufzug kann die Desinfektionseinrichtung beispielsweise Oberflächen an einem Bedienpaneel und/oder an Haltegriffen desinfizieren oder gegebenenfalls sogar den gesamten Innenraum einer Aufzugskabine.

In einer bevorzugten Ausgestaltung verfügt die Desinfektionseinrichtung hierbei über eine Lichtquelle, welche energiereiches ultraviolettes Licht emittieren kann, um Keime unschädlich zu machen. Als Lichtquelle kann beispielsweise eine oder mehrere UVC-LEDs dienen. Eine Desinfektionsintensität, mit der eine derartige Desinfektionseinrichtung Oberflächen desinfiziert, hängt hierbei maßgeblich von einer Bestrahlungsdosis ab, das heißt davon, wie groß die Lichtmenge ist, die pro Zeiteinheit auf eine vorgegebene Oberfläche trifft. Um eine ausreichende Desinfektionswirkung zu gewährleisten, sollte diese Bestrahlungsdosis beziehungsweise Desinfektionsintensität größer als eine vorab zu ermittelnde Mindest-Desinfektionsintensität sein.

Generell kann die Desinfektionseinrichtung aber auch anders ausgestaltet sein. Beispielsweise kann eine zu desinfizierende Fläche von der Desinfektionseinrichtung mit einer desinfizierend wirkenden Desinfektionsflüssigkeit wie beispielsweise einem Alkohol besprüht oder benetzt werden. Eine Desinfektionsintensität kann hierbei von einer Menge, einer Temperatur, einer chemischen Zusammensetzung und/oder anderen Parametern der aufgebrachten Desinfektionsflüssigkeit abhängen. Alternativ kann eine zu desinfizierende Fläche von der Desinfektionseinrichtung mit einem desinfizierend wirkenden Desinfektionsgas wie beispielsweise Ozon begast werden. Eine Desinfektionsintensität kann hierbei von einem Gasfluss, einer Gaskonzentration, einer Gastemperatur, einer chemischen Zusammensetzung des Gases und/oder anderen Parametern abhängen.

Die Desinfektionseinrichtung verfügt über eine Steuerung, die deren Betrieb steuert. Insbesondere können eine Desinfektionsaktivität, Desinfektionsintensität und/oder Desinfektionsart gesteuert werden. Die Desinfektionsaktivität gibt hierbei an, ob die Desinfektionseinrichtung aktuell aktiviert oder deaktiviert ist. Die Desinfektionsintensität gibt ein Maß der zu bewirkenden Desinfektionswirkung an. Die Desinfektionsart kann beispielsweise vorgeben, welcher Wirkmechanismus von der Desinfektionseinrichtung aktuell eingesetzt werden soll.

Die Steuerung der Desinfektionseinrichtung kann dabei vorzugsweise Betriebsparameter steuern, welche mit der von der Desinfektionseinrichtung zu bewirkenden Desinfektionstätigkeit korrelieren.

Beispielsweise kann die Steuerung einer Desinfektionseinrichtung, die Lichtquellen zum Emittieren von UV-Licht als desinfizierenden Wirkmechanismus einsetzt und bei der die Desinfektionsintensität überwiegend von der Lichtintensität abhängt, den Strom, der den Lichtquellen zugeführt wird, als Betriebsparameter steuern. Je stärker dieser Strom ist, umso höher ist die Lichtintensität und umso höher ist typischerweise die Desinfektionsintensität.

Prinzipiell wäre vorstellbar, dass die Desinfektionseinrichtung völlig autonom und unabhängig von anderen Komponenten der Personentransportanlage betrieben wird.

Es wurde jedoch einerseits als vorteilhaft erkannt, die Desinfektionseinrichtung angepasst an verschiedene Betriebsbedingungen der Personentransportanlage und somit bedarfsgerecht betreiben zu können.

Beispielsweise kann es gemäß einer Ausführungsform vorteilhaft sein, wenn das Steuergerät die Steuersignale unter Berücksichtigung von Informationen über eine Geschwindigkeit, mit der die Personenfördereinrichtung betrieben wird, und/oder eine Anzahl von mit der Personenfördereinrichtung beförderten Personen generiert. Generell ist davon auszugehen, dass eine hohe Anzahl von zu befördernden Personen eine erhöhte Aktivität der Desinfektionseinrichtung erforderlich machen kann. Es kann auch davon ausgegangen werden, dass bei einer höheren Geschwindigkeit der Personenfördereinrichtung die Desinfektionseinrichtung mit einer höheren Leistung betrieben werden sollte als bei niedrigeren Geschwindigkeiten, da einerseits vermutlich mehr Passagiere pro Zeiteinheit befördert werden. Andererseits ist davon auszugehen, dass für den Fall, dass die zu desinfizierende Oberfläche mit der Personenfördereinrichtung mitbewegt wird, wie dies beispielsweise bei einem Handlauf einer Fahrtreppe der Fall ist, eine Dauer, innerhalb derer diese Oberfläche von der Desinfektionseinrichtung desinfiziert wird, mit zunehmender Geschwindigkeit kürzer wird, sodass eine Desinfektion schneller durchgeführt werden sollte. Im Falle einer Aufzugsanlage muss wiederum sichergestellt werden, dass sich während des Desinfizierungsprozesses keine Person in der Aufzugskabine befindet, da die desinfizierenden Maßnahmen üblicherweise Biozid sind und somit die Gesundheit der Benutzer gefährden könnten. Insgesamt kann dadurch, dass Informationen, die beispielsweise dem Steuergerät der Personentransportanlage zur Verfügung stehen, auch beim Steuern der Desinfektionseinrichtung benutzt werden, um einen bedarfsgerechteren Betrieb der Desinfektionseinrichtung zu ermöglichen.

Andererseits wurde es auch als vorteilhaft erkannt, Informationen über einen aktuellen Status der Desinfektionseinrichtung für andere Komponenten der Personentransportanlage zugänglich zu machen und/oder solche Informationen einer externen Überwachung der Personentransportanlage bereitzustellen.

Zu diesem Zweck wird vorgeschlagen, die Steuerung der Desinfektionseinrichtung und das Steuergerät der Personenfördereinrichtung über ein bidirektionales Kommunikationssystem miteinander zu koppeln. Ein solches bidirektionales Kommunikationssystem ermöglicht eine Datenübertragung in beide entgegengesetzten Richtungen, d.h. von dem Steuergerät zu der Steuerung und von der Steuerung zu dem Steuergerät. Das bidirektionale Kommunikationssystem kann technisch auf unterschiedliche Arten implementiert werden, beispielsweise mithilfe eines Bussystems, einer Bluetooth-Kommunikation oder ähnlichem. Dabei kann es drahtgebunden oder drahtlos ausgeführt sein.

Außerdem sollen sowohl die Steuerung der Desinfektionseinrichtung als auch das Steuergerät der Personenfördereinrichtung jeweils dazu eingerichtet sein, sowohl ihrerseits Signale zu generieren und an die jeweils andere Komponente zu übertragen als auch von der anderen Komponente Signale zu empfangen.

Konkret soll die Steuerung der Desinfektionseinrichtung Statussignale generieren können, welche Informationen über einen aktuellen Betrieb, einen aktuellen Zustand und/oder eine aktuelle Konfiguration der Desinfektionseinrichtung wiedergeben. Dabei gibt die Information über den aktuellen Betrieb beispielsweise an, ob die Desinfektionseinrichtung aktuell aktiv oder deaktiviert ist. Die Information über den aktuellen Zustand kann zum Beispiel angeben, welche Soll-Eigenschaften die Desinfektionseinrichtung bzw. deren Komponenten haben und/oder welche Ist-Eigenschaften sich beispielsweise aufgrund von eingetretenem und vorzugsweise mitprotokolliertem Verschleiß mit der Zeit eingestellt haben. Der aktuelle Zustand kann beispielsweise mithilfe von Sensoren und/oder durch Analyse bisheriger Zustände und Berücksichtigen zurückliegender und aktueller Betriebsparameter und/oder Betriebsdauern ermittelt werden. Die Information über die aktuelle Konfiguration kann zum Beispiel angeben, mit welcher einer Vielzahl von möglichen Parametereinstellungen die Desinfektionseinrichtung aktuell betrieben wird. Bei einer mit Lichtquellen betriebenen Desinfektionseinrichtung kann die aktuelle Konfiguration beispielsweise eine Information über eine den Lichtquellen zugeführte Stromstärke umfassen.

Das Steuergerät der Personentransportanlage soll Steuersignale selbst generieren und/oder von anderen Quellen wie beispielsweise einem entfernt angeordneten Computer erhalten können und diese dann an die Steuerung der Desinfektionseinrichtung übermitteln können. Diese Steuersignale sollen die Steuerung anweisen, mit welchen Betriebsparametern die Desinfektionseinrichtung betrieben werden soll. Hierbei können die Steuersignale beispielsweise Sollvorgaben für einen aktuellen Betrieb und/oder eine aktuelle Konfiguration der Desinfektionseinrichtung angeben.

Aufgrund der bidirektionalen Kommunikation und des Austauschs von Informationen zwischen der Desinfektionseinrichtung und dem Steuergerät der Personentransportanlage kann somit ein aktueller Status der Desinfektionseinrichtung dem Steuergerät mitgeteilt werden und von diesem beim Steuern der Desinfektionseinrichtung und/oder anderer Komponenten der Personentransportanlage berücksichtigt werden. Die Informationen über den Status der Desinfektionseinrichtung können dabei quasi in Echtzeit berücksichtigt werden, sodass eine Art Regelung der Desinfektionseinrichtung bewirkt werden kann. Alternativ können die Informationen über eine Zeit hin akkumuliert werden, sodass beim Steuern der Desinfektionseinrichtung deren Historie wie zum Beispiel eine Dauer eines bisherigen Betriebs, dabei eingestellte Betriebsparameter (z.B. zugeführte Stromstärke), dabei vorherrschende Umgebungsbedingungen (z.B. Temperaturen), Einsatzzeiträume, etc. berücksichtigt werden können. Hierdurch kann ein effizienter, zuverlässiger und/oder bedarfsgerechter Betrieb der Desinfektionseinrichtung ermöglicht werden.

Wie bereits weiter oben erwähnt, ist die Steuerung der Desinfektionseinrichtung dazu eingerichtet, eine Desinfektionsintensität, mit der die Desinfektionseinrichtung die zu desinfizierende Oberfläche desinfiziert, abhängig von Steuersignalen, welche der Steuerung von dem Steuergerät der Personentransportanlage übersendet werden und welche einen mit einer aktuell zu bewirkenden Desinfektionsintensität korrelierenden Betriebsparameter der Desinfektionseinrichtung angeben, zu steuern.

Anders ausgedrückt soll die bereits beschriebene bidirektionale Kommunikationsfähigkeit zwischen dem Steuergerät der Personentransportanlage und der Steuerung der Desinfektionseinrichtung dazu genutzt werden, den Betrieb der Desinfektionseinrichtung hinsichtlich einer zu bewirkenden Desinfektionsintensität mithilfe von Steuersignalen, welche von dem Steuergerät der Personentransportanlage an die Steuerung der Desinfektionseinrichtung übermittelt werden zu steuern. Die Steuersignale weisen die Steuerung der Desinfektionseinrichtung dazu an, einen oder mehrere Betriebsparameter der Desinfektionseinrichtung derart einzustellen, dass sich eine gewünschte Desinfektionsintensität ergibt.

Die Desinfektionsintensität kann dabei beispielsweise als Maß definiert sein, welches angibt, wie viele Prozente einer an einer Oberfläche vorhandenen Keimbelastung in einer Zeiteinheit unschädlich gemacht werden sollen.

Beispielsweise kann bei einer Desinfektionseinrichtung mit UV-Lichtquellen ein Steuersignal angeben, welche elektrische Leistung den UV-Lichtquellen zugeführt werden soll. Die elektrische Leistung korreliert dabei mit der von den Lichtquellen emittierten Lichtintensität über die Zeit und somit letztendlich mit der bewirkten Desinfektionsintensität.

Gemäß einer konkretisierten Ausführungsform kann das Steuergerät insbesondere Steuersignale, welche einen Soll-Wert für den mit einer aktuell zu bewirkenden Desinfektionsintensität korrelierenden Betriebsparameter der Desinfektionseinrichtung angeben, unter Berücksichtigung der von der Steuerung empfangenen Statussignalen generieren.

Mit anderen Worten soll das Steuergerät beim Generieren der Steuersignale die Statussignale, welche aktuell oder in einem vorangehenden Zeitraum von der Steuerung der Desinfektionseinrichtung empfangen wurden, berücksichtigen. Dementsprechend kann die Desinfektionseinrichtung in einer Weise gesteuert werden, bei der unter anderem Informationen über deren eigenen Betrieb, Zustand und/oder Konfiguration berücksichtigt werden können. Die Steuersignale können dabei einen Soll-Wert für den Betriebsparameter, der mit der aktuell zu bewirkenden Desinfektionsintensität korreliert, angeben.

Gemäß einer Ausführungsform kann die Desinfektionseinrichtung dazu eingerichtet sein, in einem Neuzustand als Maximal-Desinfektionsintensität eine Desinfektionsintensität bewirken zu können, welche größer ist als eine für eine ausreichende Desinfizierung der zu desinfizierenden Oberfläche notwendige Mindest-Desinfektionsintensität. Das Steuergerät der Personentransportanlage und/oder die Steuerung der Desinfektionseinrichtung können hierbei dazu eingerichtet sein, die Desinfektionseinrichtung im Normalbetrieb mit einer Desinfektionsintensität oberhalb der Mindest-Desinfektionsintensität zu betreiben und einen mit einer aktuell bewirkten Desinfektionsintensität korrelierenden Betriebsparameter der Desinfektionseinrichtung sukzessive zu steigern.

Anders ausgedrückt kann die Desinfektionseinrichtung in gewissem Maße überdimensioniert sein, d.h. derart konzipiert sein, dass sie vor Eintreten von Verschleißerscheinungen eine Maximal-Desinfektionsintensität bewirken kann, die höher ist als eine Mindest-Desinfektionsintensität, die zum ausreichenden Desinfizieren einer keimbesetzten Oberfläche mindestens notwendig ist. Die Mindest-Desinfektionsintensität kann hierbei gemäß hygienischen Vorgaben definiert sein. Die überdimensionierte Maximal-Desinfektionsintensität kann beispielsweise um einen vorgegebenen Prozentsatz, beispielsweise wenigstens 10% oder wenigstens 20%, über der Mindest-Desinfektionsintensität liegen.

Es wurde erkannt, dass die tatsächlich von der Desinfektionseinrichtung bewirkte Desinfektionsintensität mit der Zeit aufgrund von Verschleißerscheinungen abnehmen kann. Beispielsweise wurde beobachtet, dass eine von einer UVC-LED emittierte Strahlungsintensität trotz gleichbleibendem Versorgungsstrom mit der Zeit abnehmen kann. Dementsprechend wurden auch bisherige Desinfektionseinrichtungen stets zu einem gewissen Grad überdimensioniert. Dabei wurden solche Desinfektionseinrichtungen typischerweise immer mit ihrer angegebenen Nennleistung betrieben, d.h., sie wurden zur Abgabe ihrer Maximal-Desinfektionsintensität angesteuert. Anfangs wurde somit eine höhere Desinfektionsintensität bewirkt, als dies zum zuverlässigen Desinfizieren nötig war. Hierdurch stellte sich sowohl ein erhöhter Energieverbrauch als auch ein erhöhter Verschleiß bei der Desinfektionseinrichtung ein.

Es wird nun vorgeschlagen, die anfängliche Überdimensionierung der Desinfektionseinrichtung dahingehend zu nutzen, dass zu Beginn eines Betriebs die Desinfektionseinrichtung mit Betriebsparametern betrieben wird, welche eine Desinfektionsintensität unterhalb der Maximal-Desinfektionsintensität bewirken. Die Desinfektionseinrichtung soll somit anfangs mit weniger als ihrer Nennleistung betrieben werden. Allerdings soll die Desinfektionseinrichtung stets derart betrieben werden, dass eine bewirkte Desinfektionsintensität oberhalb der notwendigen Mindest-Desinfektionsintensität ist.

Im Verlaufe der Zeit soll dabei der Betriebsparameter der Desinfektionseinrichtung, der mit der aktuell bewirkten Desinfektionsintensität korreliert, sukzessive gesteigert werden. Durch dieses sukzessive Steigern des Betriebsparameters können Effekte, die eine Desinfektionsintensität beispielsweise aufgrund von in der Desinfektionseinrichtung auftretendem Verschleiß mit der Zeit verringern, kompensiert werden.

Insgesamt kann hierdurch erreicht werden, dass einerseits stets eine ausreichende Desinfektionswirkung gewährleistet werden kann, andererseits die Desinfektionseinrichtung jedoch nicht mit einer übermäßig hohen Desinfektionsintensität betrieben wird, sodass Energieverbrauch und Verschleiß minimiert werden können.

Gemäß einer konkretisierten Ausführungsform können hierbei das Steuergerät der Personentransportanlage und/oder die Steuerung der Desinfektionseinrichtung dazu eingerichtet sein, den mit einer aktuell bewirkten Desinfektionsintensität korrelierenden Betriebsparameter der Desinfektionseinrichtung abhängig von einer Betriebsdauer seit Inbetriebnahme der Desinfektionseinrichtung zu steigern.

Anders ausgedrückt kann der Betriebsparameter, mit dem die Desinfektionseinrichtung betrieben wird und der mit der bewirkten Desinfektionsintensität korreliert, anfangs mit weniger als einem Nennleistung-Wert betrieben werden und dann abhängig von der Betriebsdauer, die seit Inbetriebnahme der Desinfektionseinrichtung verstrichen ist, sukzessive gesteigert werden. Die Steigerung des Betriebsparameters kann dabei in gewissen Zeitabständen von beispielsweise mehreren Tagen oder Wochen, d.h. zum Beispiel periodisch, erfolgen. Alternativ kann der Betriebsparameter mit der Zeit linear gesteigert werden. Ein solches lediglich von der bisherigen Betriebsdauer abhängiges Steigern des Betriebsparameters kann einfach und kostengünstig zu realisieren sein.

Je nach Ausmaß der anfänglichen Überdimensionierung der Desinfektionseinrichtung kann dabei bekannt sein, wie oft bzw. über welchen Zeitraum hin der Betriebsparameter gesteigert werden kann, bis die Desinfektionseinrichtung schließlich mit maximal zulässigen Betriebsparametern betrieben wird und somit kurz vor ihrem Lebensdauerende steht.

Gemäß einer zu bevorzugenden Ausführungsform kann die Desinfektionseinrichtung ferner einen Sensor zum Erfassen einer Messgröße, welche mit der Desinfektionsintensität korreliert, aufweisen. Das Steuergerät der Personentransportanlage und/oder die Steuerung der Desinfektionseinrichtung können hierbei dazu eingerichtet sein, den mit einer aktuell bewirkten Desinfektionsintensität korrelierenden Betriebsparameter der Desinfektionseinrichtung abhängig von der von dem Sensor erfassten Messgröße zu steigern.

Mit anderen Worten kann die Desinfektionseinrichtung vorzugsweise über einen Sensor verfügen, mithilfe dessen die tatsächlich von der Desinfektionseinrichtung bewirkte Desinfektionsintensität ermittelt werden kann. Die tatsächlich von der Desinfektionseinrichtung bewirkte Desinfektionsintensität braucht somit nicht mehr anhand der Betriebsparameter, mit der die Desinfektionseinrichtung betrieben wird, abgeschätzt werden, sondern kann mithilfe des Sensors tatsächlich gemessen werden. Eine von diesem Sensor erfasste Messgröße kann dann von dem Steuergerät bzw. der Steuerung berücksichtigt werden, um den mit der bewirkten Desinfektionsintensität korrelierenden Betriebsparameter geeignet anpassen zu können und mit der Zeit derart steigern zu können, dass stets eine ausreichende, aber nicht unnötig hohe, Desinfektionsintensität bewirkt wird.

Gemäß einer weiter konkretisierten Ausführungsform können das Steuergerät der Personentransportanlage und/oder die Steuerung der Desinfektionseinrichtung dazu eingerichtet sein, den mit einer aktuell bewirkten Desinfektionsintensität korrelierenden Betriebsparameter der Desinfektionseinrichtung abhängig von der von dem Sensor erfassten Messgröße zu regeln.

Anders ausgedrückt kann die von dem Sensor erfassten Messgröße, welche die tatsächlich von der Desinfektionseinrichtung bewirkte Desinfektionsintensität wiedergibt, dazu verwendet werden, um den Betrieb der Desinfektionseinrichtung durch geeignetes Einstellen des Betriebsparameters nicht nur zu steuern, sondern zu regeln. Die von dem Sensor erfasste Messgröße kann hierbei als Regelgröße in einem Regelkreis dienen.

Besonders bevorzugt kann hierbei gemäß einer Ausführungsform sein, den mit einer aktuell zu bewirkenden Desinfektionsintensität korrelierenden Betriebsparameter der Desinfektionseinrichtung kontinuierlich oder in geeigneten Zeitabständen zu überwachen und bei Übersteigen des Betriebsparameters über einen vorbestimmten Schwellenwert ein Warnsignal auszugeben, welches ein nahendes Ende einer Lebenszeit der Desinfektionseinrichtung angibt.

Anders ausgedrückt kann der Betriebsparameter, mit dem die Desinfektionseinrichtung betrieben wird und der mit der bewirkten Desinfektionsintensität korreliert, überwacht werden und geprüft werden, wie dieser sich in Relation zu einem vorbestimmten Schwellenwert verhält. Anfänglich wird die Desinfektionseinrichtung mit einem Betriebsparameter betrieben, der deutlich unterhalb dieses Schwellenwerts liegt. Durch das sukzessive Steigern des Betriebsparameters nähert dieser sich mit der Zeit dem Schwellenwert. Der Schwellenwert kann dabei kleiner oder gleich einem maximal zulässigen Wert des Betriebsparameters sein. Wenn der Schwellenwert gleich dem maximal zulässigen Wert des Betriebsparameters ist, bedeutet ein Erreichen des Schwellenwerts, dass der Betriebsparameter nicht weiter gesteigert werden sollte, da ansonsten Nachteile wie beispielsweise eine Schädigung der Desinfektionseinrichtung drohen können. Das Erreichen des Schwellenwerts entspricht damit dem Erreichen eines Lebensdauerendes der Desinfektionseinrichtung, sodass diese gewartet oder ersetzt werden sollte. Vorzugsweise wird der Schwellenwert geringfügig niedriger als der maximal zulässige Wert des Betriebsparameters gewählt. Dementsprechend bedeutet ein Erreichen des Schwellenwerts dann, dass das Lebensdauer Ende der Desinfektionseinrichtung zwar zeitnah bevorsteht, aber vorzugsweise ausreichend Zeit für Maßnahmen wie die Wartung oder den Austausch der Desinfektionseinrichtung verbleibt.

Bei Erreichen des Schwellenwerts kann ein entsprechendes Warnsignal ausgegeben werden. Dieses Warnsignal kann beispielsweise an das Steuergerät der Personentransportanlage übermittelt werden. Dort kann das Warnsignal beispielsweise in einer für einen Techniker wahrnehmbaren Weise ausgegeben werden. Alternativ kann das Warnsignal von dem Steuergerät an eine externe Überwachungsvorrichtung weitergeleitet werden und dort z.B. das Einleiten geeigneter Wartungs- oder Ersetzungsmaßnahmen auslösen.

Gemäß einer Ausführungsform weist die Desinfektionseinrichtung ferner einen Sensor zum Erfassen einer Temperatur innerhalb der Desinfektionseinrichtung und/oder zum Erfassen einer Temperatur in einer Umgebung angrenzend an die Desinfektionseinrichtung auf. Das Steuergerät der Personentransportanlage und/oder die Steuerung der Desinfektionseinrichtung sind hierbei dazu eingerichtet, den Betrieb der Desinfektionseinrichtung abhängig von der von dem Sensor ermittelten Temperatur zu steuern.

Es wurde erkannt, dass manche Desinfektionseinrichtungen die von Ihnen erzeugten Desinfektionswirkungen abhängig von Temperaturbedingungen ändern können oder bei bestimmten Temperaturbedingungen sogar geschädigt werden. Beispielsweise wurde erkannt, dass UVC-LEDs nicht oberhalb bestimmter Temperaturgrenzen betrieben werden sollten, da sie ansonsten Schaden nehmen.

Es wird daher vorgeschlagen, die Desinfektionseinrichtung mit einem Sensor auszustatten, mithilfe dessen die Temperatur der Desinfektionseinrichtung bzw. einzelner Komponenten derselben und/oder eine Umgebungstemperatur ermittelt werden kann. Ein diese Temperatur repräsentierendes Sensorsignal kann dann von der Steuerung der Desinfektionseinrichtung und/oder dem Steuergerät der Personentransportanlage beim Steuern des Betriebs der Desinfektionseinrichtung berücksichtigt werden. Beispielsweise kann bei erhöhten gemessenen Temperaturen eine Leistungszufuhr zu der Desinfektionseinrichtung reduziert bzw. begrenzt werden, um einer weiteren Erwärmung entgegenzuwirken.

Bezüglich der vorangehend beschriebenen Ausgestaltung der Desinfektionseinrichtung mit einem Sensor zum Erfassen einer mit der Desinfektionsintensität korrelierenden Messgröße und/oder einem Temperatursensor kann gemäß einer Ausführungsform die Steuerung der Desinfektionseinrichtung dazu eingerichtet sein, die von dem Sensor ermittelte Messgröße und/oder ein mit der von dem Sensor ermittelten Messgröße korrelierendes Datensignal an das Steuergerät der Personentransportanlage zu senden.

Anders ausgedrückt kann die Steuerung der Desinfektionseinrichtung vorzugsweise nicht nur Statussignale betreffend den aktuellen Betrieb, Zustand oder Konfiguration der Desinfektionseinrichtung an das Steuergerät der Personentransportanlage übermitteln, sondern ergänzend auch Messgrößen von in der Desinfektionseinrichtung integrierten Sensoren dem Steuergerät zur Verfügung stellen. Dabei kann entweder die von dem Sensor ermittelte Messgröße selbst oder ein durch Verarbeiten dieser Messgröße ermitteltes Datensignal an das Steuergerät gesendet werden.

Das Steuergerät kann die darin enthaltene Information dann beispielsweise selbst verarbeiten und beim Steuern der Desinfektionseinrichtung oder anderer Komponenten verwenden. Alternativ oder ergänzend kann das Steuergerät die entsprechenden Daten für eine spätere Verwendung speichern und/oder an andere Komponenten weitergeben. Insbesondere können die Daten beispielsweise an einen externen Computer weitergeleitet werden, der zum Beispiel Teil einer entfernt angeordneten Überwachungseinrichtung oder einer Datenwolke ("Cloud") ist. Dort können die ermittelten Messgrößen überwacht und/oder verarbeitet werden.

Gemäß einer Ausführungsform kann die Personentransportanlage hierzu eine Schnittstelleneinrichtung aufweisen, welche dazu eingerichtet ist, Daten von dem Steuergerät der Personentransportanlage an den entfernt angeordneten Computer zu senden und/oder Daten von dem entfernt angeordneten Computer für einen Empfang durch das Steuergerät der Personentransportanlage bereitzustellen.

Mit anderen Worten kann die Personentransportanlage mithilfe der Schnittstelleneinrichtung über die Möglichkeit verfügen, Daten oder Signale mit einem entfernt angeordneten Computer auszutauschen. Der entfernt angeordnete Computer kann sich außerhalb des Gebäudes, in dem die Personentransportanlage installiert ist, befinden. Insbesondere kann der entfernt angeordneten Computer Teil einer externen Überwachungseinrichtung sein, mit der die Personentransportanlage bzw. deren Betrieb überwacht werden sollen. Alternativ kann der Computer Teil einer Datenwolke sein, in der beispielsweise Informationen betreffend die Personentransportanlage gespeichert sein können. Das Steuergerät der Personentransportanlage kann hierbei insbesondere Signale und Daten, welches es zuvor von der Steuerung der Desinfektionseinrichtung erhalten hat, über die Schnittstelleneinrichtung an den externen Computer weiterleiten.

Gemäß einer Ausführungsform des Verfahrens zum Betreiben der hierin vorgeschlagenen Personentransportanlage kann in dem entfernt angeordneten Computer beispielsweise ein digitaler Zwilling der Personentransportanlage gespeichert sein. Eigenschaften der Personentransportanlage können dann basierend auf dem digitalen Zwilling der Personentransportanlage unter Berücksichtigung der von der Steuerung der Desinfektionseinrichtung übermittelten Statussignale ermittelt werden.

Die Formulierung "digitaler Zwilling" kann hierbei für einen Datensatz stehen, in dem physikalische und/oder funktionale Eigenschaften der Personentransportanlage möglichst realitätsnah wiedergegeben sind. Beispielsweise kann der digitale Zwilling Angaben über geometrische, elektrische, magnetische, thermische und/oder andere Eigenschaften der Personentransportanlage enthalten. Basierend auf diesen Daten können aktuelle oder gegebenenfalls auch zukünftige Eigenschaften der Personentransportanlage berechnet, simuliert oder modelliert werden.

Es wird nun vorgeschlagen, die Statussignale, die aktuelle Eigenschaften betreffend die Desinfektionseinrichtung wiedergeben, ergänzend in den digitalen Zwilling einzupflegen. Dementsprechend können Eigenschaften der Personentransportanlage unter Verwendung des digitalen Zwillings noch genauer ermittelt werden, indem dabei auch Eigenschaften der Desinfektionseinrichtung berücksichtigt werden.

Beispielsweise kann anhand von Informationen, die Aufschluss über eine Desinfektionsintensität und/oder Desinfektionsaktivität während eines zurückliegenden Betriebszeitraums der Personentransportanlage geben, auf einen hierdurch bedingten Einfluss auf andere Komponenten der Personentransportanlage rückgeschlossen werden.

Konkret kann in einem Beispiel, bei dem die Desinfektionseinrichtung die Desinfektionswirkung mithilfe von UVC-LEDs erzeugt, eine Information über Betriebsdauern und/oder Beleuchtungsintensitäten durch Einpflegen dieser Information in einen digitalen Zwilling einen Rückschluss beispielsweise darüber ermöglichen, inwieweit hierbei emittierte UVC-Strahlung andere Komponenten der Personentransportanlage wie beispielsweise ein Gewebe und/oder einen Kunststoff eines Handlaufs einer Fahrtreppe mit der Zeit geschädigt haben könnte.

Gemäß einer weiteren Ausführungsform kann die Desinfektionseinrichtung ferner eine Ausgabeeinrichtung aufweisen, welche dazu eingerichtet ist, ein für einen Menschen wahrnehmbares Signal auszugeben, welches einen aktuellen Zustand der Desinfektionseinrichtung angibt.

Mit anderen Worten kann direkt an der Desinfektionseinrichtung eine Ausgabeeinrichtung beispielsweise in Form einer Lichtquelle, einer Anzeige, eines Lautsprechers oder einer ähnlichen technischen Komponente vorgesehen sein, mithilfe derer ein für Menschen optisch, akustisch oder in anderer Weise wahrnehmbares Signal ausgegeben werden kann.

Diese Ausgabeeinrichtung kann genutzt werden, um beispielsweise einem Wartungstechniker eine Information über den aktuellen Zustand der Desinfektionseinrichtung übermitteln zu können. Der Wartungstechniker kann somit direkt vor Ort und in einfacher Weise feststellen, ob die Desinfektionseinrichtung erwartungsgemäß funktioniert oder beispielsweise gewartet oder ausgetauscht werden muss. Die Ausgabeeinrichtung kann aber auch ein Bildschirm oder eine optische Anzeige sein, die gut sichtbar für Benutzer der Personentransportanlage angeordnet ist und ihnen anzeigt, dass die Desinfektionseinrichtung funktioniert und dass sie deshalb desinfizierte Handläufe erwarten können.

Es wird darauf hingewiesen, dass einige der möglichen Merkmale und Vorteile der Erfindung hierin mit Bezug auf unterschiedliche Ausführungsformen einer Personentransportanlage einerseits und eines Verfahrens zum Betreiben dieser Transportanlage andererseits beschrieben sind. Ein Fachmann erkennt, dass die Merkmale in geeigneter Weise kombiniert, angepasst oder ausgetauscht werden können, um zu weiteren Ausführungsformen der Erfindung zu gelangen.

Nachfolgend werden Ausführungsformen der Erfindung unter Bezugnahme auf die beigefügte Zeichnung beschrieben, wobei weder die Zeichnung noch die Beschreibung als die Erfindung einschränkend auszulegen sind.

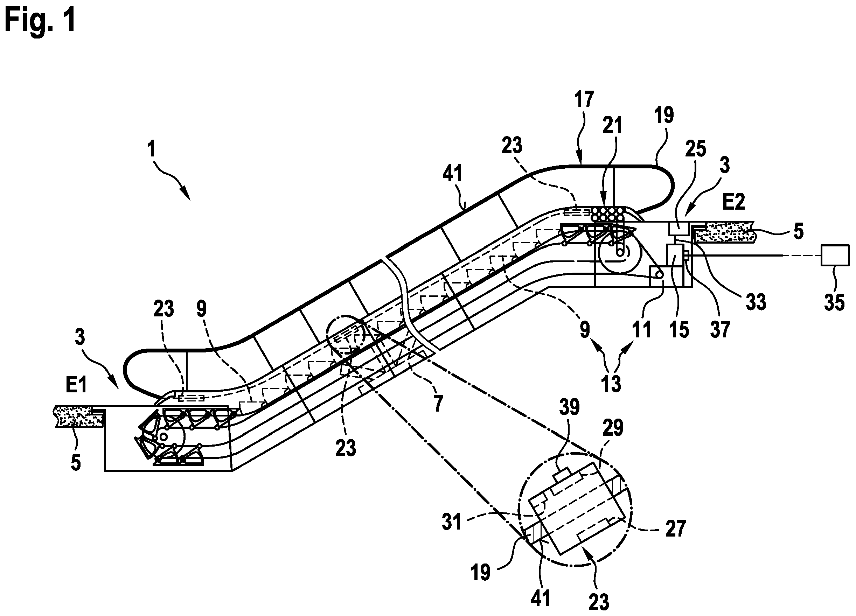

Fig. 1 zeigt eine vereinfachte Ansicht einer als Fahrtreppe ausgestalteten Personentransportanlage mit einer Handlauf-Desinfektionseinrichtung.

Die Figur ist lediglich schematisch und nicht maßstabsgetreu. Gleiche Bezugszeichen bezeichnen gleiche oder gleichwirkende Merkmale

Figur 1 zeigt eine vereinfachte Ansicht einer Personentransportanlage 1. Die als Fahrtreppe ausgestaltete Personentransportanlage 1 verbindet eine untere Ebene E1 mit einer oberen Ebene E2 eines Bauwerks 5. Die Personentransportanlage 1 kann über Zutrittsbereiche 3 betreten und wieder verlassen werden. In einem Tragwerk 7 ist ein umlaufendes Stufenband 9 angeordnet, welches in der oberen Ebene E2 und in der unteren Ebene E1 umgelenkt wird und somit einen vorlaufenden Abschnitt und einen rücklaufenden Abschnitt aufweist. Der besseren Übersicht wegen wurde auf die detaillierte Darstellung des rücklaufenden Abschnitts verzichtet, ebenso auch auf eine detaillierte Darstellung von Spanten, Führungsschienen, und Schienenblöcken. Die Personentransportanlage 1 verfügt über eine Antriebseinheit 11, um das Stufenband 9 anzutreiben. Zusammen bilden das Stufenband 9 und die Antriebseinheit 11 eine Personenfördereinrichtung 13, mittels derer Passagiere in dem Bauwerk 5 zwischen den Zutrittsbereichen 3 befördert werden können. Ein Steuergerät 15 dient dazu, den Betrieb der Personenfördereinrichtung 13 zu steuern.

Die Personentransportanlage 1 weist ferner zwei Balustraden 17 auf, die sich entlang jeder Längsseite des Stufenbandes 9 erstrecken, wobei in Figur 1 nur die in der Betrachtungsebene im Vordergrund angeordnete Balustrade 17 sichtbar ist. An jeder Balustrade 17 ist ein Handlauf 19 umlaufend angeordnet, wobei dessen rücklaufendes Trum in einem Balustradensockel 21 geführt ist. Dieser Balustradensockel 21 verbindet die Balustrade 17 mit dem Tragwerk 7.

Des Weiteren weist die Personentransportanlage 1 für jeden umlaufenden Handlauf 19 mindestens eine Desinfektionseinrichtung 23 auf. Diese ist beispielsweise ebenfalls im Balustradensockel 21 der Balustrade 17 installiert und damit vor den Benutzern der Personentransportanlage 1 verdeckt. Ein Betrieb der Desinfektionseinrichtung 23 wird von einer Steuerung 25 gesteuert.

Im dargestellten Beispiel kann die Desinfektionseinrichtung 23 mit einer oder mehreren UVC-LEDs 27 ausgestattet sein. Mithilfe des von diesen UVC-LEDs 27 emittierten UVC-Lichts kann eine Teiloberfläche des Handlaufs 19 bestrahlt werden, wobei das hochenergetische UVC-Licht dort befindliche Keime abtöten kann. Die Steuerung 25 kann unter anderem eine Stromversorgung der Desinfektionseinrichtung 23 bzw. von deren UVC-LEDs 27 steuern.

Die Desinfektionseinrichtung 23 kann ferner über einen oder mehrere Sensoren 29, 31 verfügen, mithilfe derer Betriebsbedingungen und/oder Umgebungsbedingungen erfasst werden können. Alternativ können solche Sensoren auch separat vorgesehen sein und Signale an die Desinfektionseinrichtung 23 übermitteln. Beispielsweise kann die Desinfektionseinrichtung 23 über einen UVC-Sensor 29 verfügen, dessen Messsignale angeben, welche Strahlungsintensität an UVC-Licht aktuell tatsächlich von den UVC-LEDs 27 emittiert wird und auf den UVC-Sensor 29 trifft. Ferner kann ein Temperatursensor 31 vorgesehen sein, mithilfe dessen eine aktuelle Temperatur der Desinfektionseinrichtung 23 bzw. von Komponenten derselben, insbesondere der UVC-LEDs 27, und/oder eine Temperatur der Umgebung der Desinfektionseinrichtung 23 gemessen werden kann.

Die Desinfektionseinrichtung 23, bzw. deren Steuerung 25, und das Steuergerät 15 der Personentransportanlage 1 sind über ein bidirektionales Kommunikationssystem 33 miteinander verbunden. In Figur 1 ist das bidirektionale Kommunikationssystem 33 als einfache Leitung veranschaulicht. Es kann in der praktischen Umsetzung jedoch auch Teil eines drahtgebundenen Bussystems oder einer drahtlosen Datenkommunikationseinrichtung sein.

Die Steuerung 25 der Desinfektionseinrichtung 23 ist dazu eingerichtet, Statussignale, welche eine Information über den aktuellen Betrieb, den aktuellen Zustand oder die aktuelle Konfiguration der Desinfektionseinrichtung 23 wiedergeben, über das Kommunikationssystem 33 an das Steuergerät 15 zu übermitteln. Übermittelte Signale können zum Beispiel eine Aktivität der Desinfektionseinrichtung 23 angeben, d.h., ob bzw. wie lange die Desinfektionseinrichtung 23 betrieben wird. Alternativ oder ergänzend können übermittelte Signale eine Information über Bedingungen, wie sie aktuell in der Desinfektionseinrichtung 23 herrschen, angeben, d.h. beispielsweise angeben, welche Temperatur von dem Temperatursensor 31 aktuell gemessen wird. Als weitere Alternative oder Ergänzung können übermittelte Signale angeben, wie Komponenten der Desinfektionseinrichtung 23 aktuell konfiguriert sind, d.h. beispielsweise mit welcher Leistung aktuell die UVC-LED 27 betrieben wird.

Die von der Desinfektionseinrichtung 23 übermittelten Statussignale können von dem Steuergerät 15 empfangen und dort verarbeitet werden. Beispielsweise kann das Steuergerät 15 die empfangenen Statussignale beim Steuern von Funktionen der Personentransportanlage 1 berücksichtigen.

Insbesondere kann das Steuergerät 15 auch Funktionen der Desinfektionseinrichtung 23 steuern, indem es geeignete Steuersignale über das Kommunikationssystem 33 an die Steuerung 25 der Desinfektionseinrichtung 23 übermittelt. Dabei können unter anderem die in den zuvor empfangenen Statussignalen enthaltenen Informationen berücksichtigt werden. Hierdurch kann ermöglicht werden, dass die Desinfektionseinrichtung 23 beispielsweise unter Berücksichtigung von darin aktuell herrschenden Temperaturen und/oder unter Berücksichtigung von sich beispielsweise aufgrund von Verschleiß mit der Zeit verändernden Eigenschaften der Desinfektionseinrichtung 23 gesteuert werden kann. Ferner kann das Steuergerät 15 beim Steuern der Desinfektionseinrichtung 23 auch Informationen betreffend andere Komponenten der Personentransportanlage 1 berücksichtigen. Beispielsweise kann das Steuergerät 15 den Betrieb der Desinfektionseinrichtung 23 derart steuern, dass einem aktuellen Passagieraufkommen und/oder einer aktuellen Geschwindigkeit, mit der die Personenfördereinrichtung 13 bzw. der Handlauf 19 verlagert wird, Rechnung getragen wird.

Vorteilhafterweise kann die Desinfektionseinrichtung 23 derart konzipiert sein, dass sie zumindest in ihrem Neuzustand in einer Weise betrieben werden kann, dass sie mehr als eine Mindest-Desinfektionsintensität, die mindestens benötigt wird, um eine zu desinfizierende Oberfläche 41 zuverlässig zu desinfizieren, leisten kann. Eine mit UVC-LEDs 27 ausgestattete Desinfektionseinrichtung 23 kann hinsichtlich der darin verbauten UVC-LEDs 27 beispielsweise derart konzipiert sein, dass es für eine Mindest-Desinfektionsintensität bereits genügen würde, die UVC-LEDs 27 mit beispielsweise nur 80 % ihrer Nennleistung zu betreiben. Die Nennleistung ist hierbei diejenige Leistung, die den UVC-LEDs 27 maximal zugeführt werden darf, ohne eine plötzliche bzw. übermäßige Schädigung derselben zu riskieren.

Eine derart konzipierte Desinfektionseinrichtung 23 wird dann vorzugsweise zumindest anfangs unterhalb ihrer Nennleistung betrieben. Anders ausgedrückt wird die Desinfektionseinrichtung 23 zwar mit einer Desinfektionsintensität betrieben, die oberhalb der Mindest-Desinfektionsintensität, aber unterhalb der maximal erreichbaren Maximal-Desinfektionsintensität liegt.

Eine Leistungsversorgung innerhalb der Desinfektionseinrichtung 23, das heißt beispielsweise eine den UVC-LEDs 27 zugeführte Stromstärke, korreliert typischerweise mit einer aktuell bewirkten Desinfektionsintensität. Anfangs wird diese Leistungsversorgung vorzugsweise niedriger als die Nennleistung eingestellt. Hierdurch kann ein Energieverbrauch sowie gegebenenfalls ein Verschleiß an der Desinfektionseinrichtung 23 und/oder an damit desinfizierten Oberflächen 41 geringgehalten werden.

Da jedoch bekannt ist, dass beispielsweise UVC-LEDs 27 mit der Zeit aufgrund von Verschleißerscheinungen bei gleicher Leistungsversorgung weniger UVC-Licht emittieren, kann die Leistungsversorgung der UVC-LEDs 27 sukzessive gesteigert werden. Beispielsweise kann die Leistungsversorgung abhängig von einer Betriebsdauer seit Inbetriebnahme der Desinfektionseinrichtung 23 sukzessive in regelmäßigen Zeitabständen oder linear gesteigert werden. Sobald die Leistungsversorgung sich der Nennleistung nähert bzw. diese erreicht, bedeutet dies, dass die Desinfektionseinrichtung 23 bzw. konkret deren UVC-LEDs 27 ihre Betriebslebensdauer erreicht haben und vermutlich ausgetauscht werden müssen.

Da verschiedene Einflüsse dazu führen können, dass sich die von der Desinfektionseinrichtung 23 tatsächlich bewirkte Desinfektionsintensität nicht in eindeutiger Weise mit der Zeit verändert, kann es besonders bevorzugt sein, die tatsächlich bewirkte Desinfektionsintensität beispielsweise mithilfe des UVC-Sensors 29 zu überwachen. Messsignale des UVC-Sensors 29 können dann von dem Steuergerät 15 berücksichtigt werden. Beispielsweise kann die Desinfektionseinrichtung 23 für den Fall, dass eine gemessene UVC-Lichtintensität auf weniger als ein für die Mindest-Desinfektionsintensität notwendiges Maß zu sinken droht, mit einer geeignet höheren Leistungsversorgung angesteuert werden.

Dabei kann es vorteilhaft sein, die tatsächlich bewirkte Leistungsversorgung, das heißt allgemein ausgedrückt den Betriebsparameter, der mit der aktuell zu bewirkenden Desinfektionsintensität korreliert, zu überwachen und diesen beispielsweise mit einem vorbestimmten Schwellenwert zu vergleichen. Konkret kann eine den UVC-LEDs 27 zugeführte elektrische Stromstärke mit einem Stromstärken-Schwellenwert verglichen werden. Der Schwellenwert kann dabei derart gesetzt sein, dass er der Nennleistung der UVC-LEDs 27 entspricht oder um ein vorgegebenes Maß unterhalb dieser Nennleistung liegt. Nähert sich die tatsächlich bewirkte Leistungsversorgung der Nennleistung und erreicht dabei den Schwellenwert, kann dies die Ausgabe eines Warnsignals veranlassen, welches ein nahendes Ende der Lebenszeit der Desinfektionseinrichtung 23 angibt. Ein Techniker kann daraufhin die Desinfektionseinrichtung 23 warten bzw. austauschen. Dabei wird eine vorausschauende Wartung (englisch: predictive maintenance) ermöglicht, da das Warnsignal bereits ausreichend rechtzeitig generiert werden kann, bevor es tatsächlich zu einem Ausfall der Desinfektionseinrichtung 23 kommt, bei dem entweder deren UVC-LEDs 27 aufgrund einer übermäßigen Leistungszufuhr geschädigt wird oder bei Zuführen der Nennleistung nicht mehr die erforderliche Mindest-Desinfektionsintensität bewirkt werden kann.

Die von der Steuerung 25 der Desinfektionseinrichtung 23 übermittelten Statussignale können neben einer Nutzung direkt in dem Steuergerät 15 der Personentransportanlage 1 auch an anderer Stelle vorteilhaft genutzt werden. Beispielsweise kann das Steuergerät 15 ohnehin mithilfe einer Schnittstelleneinrichtung 37 dazu konzipiert sein, aktuelle Betriebsdaten der Personentransportanlage 1 an einen entfernt angeordneten Computer 35 weiterzugeben. Dieser entfernt angeordnete Computer 35 kann Teil einer Überwachungseinrichtung oder einer Datenwolke sein, die dazu eingesetzt werden, Funktionalitäten der Personentransportanlage 1 aus der Ferne zu überwachen. Die Statussignale der Desinfektionseinrichtung 23 können dann ebenfalls an einen solchen entfernt angeordneten Computer 35 übermittelt werden. Dort können sie beispielsweise dazu eingesetzt werden, Funktionalitäten und/oder einen Betriebszustand der Desinfektionseinrichtung 23 zu überwachen.

Der entfernt angeordnete Computer 35 kann ferner dazu eingesetzt werden, Funktionalitäten und/oder Betriebszustände der Personentransportanlage 1 bzw. konkret von deren Desinfektionseinrichtung 23 aus der Ferne zu steuern. Hierzu kann der entfernt angeordnete Computer 35 geeignete Steuersignale an das Steuergerät 15 übermitteln, welches darauf basierend wiederum Steuersignale an die Steuerung 25 der Desinfektionseinrichtung 23 senden kann.

In einer speziellen Ausgestaltung kann auf dem entfernt angeordneten Computer 35 ein Datensatz eines digitalen Zwillings gespeichert bzw. gepflegt werden. Der digitale Zwilling gibt hierbei strukturelle, physikalische und/oder funktionale Eigenschaften der Personentransportanlage 1 und insbesondere der darin verbauten Komponenten an. Die Statussignale der Desinfektionseinrichtung 23 können in diesem Fall genutzt werden, um den digitalen Zwilling zu pflegen. Zum Beispiel können in dem digitalen Zwilling gespeicherte Daten unter Berücksichtigung der in den Statussignalen enthaltenen Informationen über den aktuellen Betrieb, den aktuellen Zustand oder die aktuelle Konfiguration der Desinfektionseinrichtung 23 aktualisiert werden. Auf diese Weise können in dem digitalen Zwilling stets aktuelle Informationen betreffend die Desinfektionseinrichtung 23 enthalten sein. Ferner wird auch ermöglicht, einen von der Desinfektionseinrichtung 23 bewirkten Einfluss auf andere Komponenten oder Parameter der Personentransportanlage 1 mithilfe des aktuell gehaltenen digitalen Zwillings zu analysieren, modellieren oder simulieren. Beispielsweise kann hierdurch auf einen Einfluss, den von der Desinfektionseinrichtung 23 emittiertes UVC-Licht auf andere Komponenten wie insbesondere die zu desinfizierende Oberfläche 41 beispielsweise des Handlaufs 19 haben kann, rückgeschlossen werden.

Um einem Wartungstechniker vor Ort eine Wartung der Personentransportanlage 1 zu vereinfachen, kann diese ferner eine Ausgabeeinrichtung 39 aufweisen, welche eine Information über den aktuellen Zustand der Desinfektionseinrichtung 23 in einer für den Wartungstechniker wahrnehmbaren Weise ausgeben kann. Beispielsweise kann hierfür eine im Sichtbaren Spektrum emittierende LED vorgesehen sein. Je nachdem, welches Signal von der Ausgabeeinrichtung 39 ausgegeben wird, d.h. beispielsweise mit welcher Farbe und/oder Blinksequenz deren LED betrieben wird, kann dies dem Wartungstechnikers signalisieren, dass
(i) die Desinfektionseinrichtung 23 einwandfrei arbeitet (z.B. permanentes Leuchten der LED),
(ii) die Desinfektionseinrichtung 23 zwar noch arbeitet, sich aber ihrem Lebensdauerende zuneigt (z.B. langsames Blinken der LED),
(iii) die Desinfektionseinrichtung 23 ihr Lebensdauerende bereits erreicht hat (z.B. schnelles Blinken der LED), oder
(iv) die Desinfektionseinrichtung 23 aktuell abgeschaltet ist (z.B. LED aus).

Die Ausgabeeinrichtung 39 kann aber auch ein Bildschirm oder eine optische Anzeige sein, die gut sichtbar für Benutzer der Personentransportanlage 1 angeordnet ist und ihnen anzeigt, dass die Desinfektionseinrichtung 23 funktioniert und dass sie deshalb desinfizierte Handläufe 19 erwarten können.

Zusammenfassend kann durch den hierin vorgeschlagenen bidirektionalen Signalaustausch zwischen der Steuerung 25 der Desinfektionseinrichtung 23 und dem Steuergerät 15 der Personentransportanlage 1 die Desinfektionseinrichtung 23 bedarfsgerecht betrieben werden sowie Informationen betreffend die Desinfektionseinrichtung 23 von dem Steuergerät 15 der Personentransportanlage 1 verarbeitet und genutzt werden, beispielsweise um einen Verschleiß der Desinfektionseinrichtung 23 erkennen und Gegenmaßnahmen frühzeitig planen zu können. Ferner kann die Desinfektionseinrichtung 23 von einem entfernt angeordneten Computer 35 aus über dessen Anbindung an das Steuergerät 15 gesteuert werden oder Informationen betreffend die Desinfektionseinrichtung 23 an den entfernt angeordneten Computer 35 weitergeleitet und dort beispielsweise zur Pflege beispielsweise eines digitalen Zwillings der Personentransportanlage 1 genutzt werden.

Abschließend ist darauf hinzuweisen, dass Begriffe wie "aufweisend", "umfassend", etc. keine anderen Elemente oder Schritte ausschließen und Begriffe wie "eine" oder "ein" keine Vielzahl ausschließen. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Desinfektionseinrichtung (23) zum Desinfizieren einer Oberfläche (41) an einer Personenfördereinrichtung (13), welche Personenfördereinrichtung (13) Teil einer Personentransportanlage (1) ist und zum Befördern von Personen innerhalb eines Bauwerks (5) dient;
wobei die Desinfektionseinrichtung (23) eine Steuerung (25) zum Steuern eines Betriebs der Desinfektionseinrichtung (23) aufweist;
wobei die Steuerung (25) der Desinfektionseinrichtung (23) dazu eingerichtet ist, über ein bidirektionales Kommunikationssystem (33) an ein Steuergerät (15) der Personentransportanlage (1) angebunden zu werden, und
wobei die Steuerung (25) der Desinfektionseinrichtung (23) dazu eingerichtet ist, Statussignale, welche Informationen über einen aktuellen Betrieb, einen aktuellen Zustand und/oder eine aktuelle Konfiguration der Desinfektionseinrichtung (23) wiedergeben, an das Steuergerät (15) zu senden und von dem Steuergerät (15) der Personentransportanlage (1) unter Berücksichtigung empfangener Statussignale generierte Steuersignale zu empfangen, wobei die Steuerung (25) den Betrieb der Desinfektionseinrichtung (23) unter Berücksichtigung empfangener Steuersignale steuert, **dadurch gekennzeichnet, dass** die Steuerung (25) der Desinfektionseinrichtung (23) dazu eingerichtet ist, eine Desinfektionsintensität, mit der die Desinfektionseinrichtung (23) die zu desinfizierende Oberfläche (41) desinfiziert, abhängig von Steuersignalen, welche der Steuerung (25) von dem Steuergerät (15) der Personentransportanlage (1) übersendet werden und die einen mit einer aktuell zu bewirkenden Desinfektionsintensität korrelierenden Betriebsparameter der Desinfektionseinrichtung (23) angeben, zu steuern.

2. Personentransportanlage (1) aufweisend:
- eine Personenfördereinrichtung (13) zum Befördern von Personen innerhalb eines Bauwerks (5);
- ein Steuergerät (15) zum Steuern eines Betriebs der Personenfördereinrichtung (13); und
- eine Desinfektionseinrichtung (23) nach Anspruch 1 zum Desinfizieren einer zu desinfizierenden Oberfläche (41) an der Personenfördereinrichtung (13);
wobei die Desinfektionseinrichtung (23) eine Steuerung (25) zum Steuern eines Betriebs der Desinfektionseinrichtung (23) aufweist;
wobei die Steuerung (25) der Desinfektionseinrichtung (23) über ein bidirektionales Kommunikationssystem (33) an das Steuergerät (15) der Personentransportanlage (1) angebunden ist, und
wobei das Steuergerät (15) der Personentransportanlage (1) dazu eingerichtet ist, Statussignale, welche Informationen über einen aktuellen Betrieb, einen aktuellen Zustand und/oder eine aktuelle Konfiguration der Desinfektionseinrichtung (23) wiedergeben, von der Steuerung (25) zu empfangen und das Steuergerät (15) unter Berücksichtigung empfangener Statussignale Steuersignale generiert und an die Steuerung (25) übermittelt, wobei die Steuerung (25) den Betrieb der Desinfektionseinrichtung (23) unter Berücksichtigung empfangener Steuersignale steuert.

3. Personentransportanlage nach Anspruch 2,
wobei das Steuergerät (15) Steuersignale, welche einen Sollwert für den mit einer aktuell zu bewirkenden Desinfektionsintensität korrelierenden Betriebsparameter der Desinfektionseinrichtung (23) angeben, unter Berücksichtigung der von der Steuerung (25) empfangenen Statussignale generiert.

4. Personentransportanlage nach einem der Ansprüche 2 oder 3,
wobei die Desinfektionseinrichtung (23) dazu eingerichtet ist, in einem Neuzustand als Maximal-Desinfektionsintensität eine Desinfektionsintensität bewirken zu können, welche größer ist als eine für eine ausreichende Desinfizierung der zu desinfizierenden Oberfläche (41) notwendige Mindest-Desinfektionsintensität, und
wobei das Steuergerät (15) der Personentransportanlage (1) und/oder die Steuerung (25) der Desinfektionseinrichtung (23) dazu eingerichtet sind, die Desinfektionseinrichtung (23) im Normalbetrieb mit einer Desinfektionsintensität oberhalb der Mindest-Desinfektionsintensität zu betreiben und einen mit einer aktuell bewirkten Desinfektionsintensität korrelierenden Betriebsparameter der Desinfektionseinrichtung (23) sukzessive zu steigern.

5. Personentransportanlage nach einem der Ansprüche 2 bis 4,
wobei das Steuergerät (15) der Personentransportanlage (1) und/oder die Steuerung (25) der Desinfektionseinrichtung (23) dazu eingerichtet sind, den mit einer aktuell bewirkten Desinfektionsintensität korrelierenden Betriebsparameter der Desinfektionseinrichtung (23) abhängig von einer Betriebsdauer seit Inbetriebnahme der Desinfektionseinrichtung (23) zu steigern.

6. Personentransportanlage nach einem der Ansprüche 2 bis 5,
wobei die Desinfektionseinrichtung (23) ferner einen Sensor (29) zum Erfassen einer Messgröße, welche mit der Desinfektionsintensität korreliert, aufweist und
wobei das Steuergerät (15) der Personentransportanlage (1) und/oder die Steuerung (25) der Desinfektionseinrichtung (23) dazu eingerichtet sind, den mit einer aktuell bewirkten Desinfektionsintensität korrelierenden Betriebsparameter der Desinfektionseinrichtung (23) abhängig von der von dem Sensor (29) erfassten Messgröße zu steigern.

7. Personentransportanlage nach Anspruch 6,
wobei das Steuergerät (15) der Personentransportanlage (1) und/oder die Steuerung (25) der Desinfektionseinrichtung (23) dazu eingerichtet sind, den mit einer aktuell bewirkten Desinfektionsintensität korrelierenden Betriebsparameter der Desinfektionseinrichtung (23) abhängig von der von dem Sensor (29) erfassten Messgröße zu regeln.

8. Personentransportanlage nach einem der Ansprüche 2 bis 7,
wobei der mit einer aktuell zu bewirkenden Desinfektionsintensität korrelierende Betriebsparameter der Desinfektionseinrichtung (23) überwacht wird und bei Übersteigen des Betriebsparameters über einen vorbestimmten Schwellenwert ein Warnsignal ausgegeben wird, welches ein nahendes Ende einer Lebenszeit der Desinfektionseinrichtung (23) angibt.

9. Personentransportanlage nach einem der Ansprüche 2 bis 8,
wobei die Desinfektionseinrichtung (23) ferner einen Sensor (31) zum Erfassen einer Temperatur innerhalb der Desinfektionseinrichtung (23) und/oder zum Erfassen einer Temperatur in einer Umgebung angrenzend an die Desinfektionseinrichtung (23) aufweist, und
wobei das Steuergerät (15) der Personentransportanlage (1) und/oder die Steuerung (25) der Desinfektionseinrichtung (23) dazu eingerichtet sind, den Betrieb der Desinfektionseinrichtung (23) abhängig von der von dem Sensor (31) ermittelten Temperatur zu steuern.

10. Personentransportanlage nach Anspruch 6 oder 9,
wobei die Steuerung (25) der Desinfektionseinrichtung (23) dazu eingerichtet ist, die von dem Sensor (29, 31) ermittelte Messgröße und/oder ein mit der von dem Sensor (29, 31) ermittelten Messgröße korrelierendes Datensignal an das Steuergerät (15) der Personentransportanlage (1) zu senden.

11. Personentransportanlage nach einem der Ansprüche 2 bis 10,
wobei die Personentransportanlage (1) ferner eine Schnittstelleneinrichtung (37) aufweist, welche dazu eingerichtet ist, Daten von dem Steuergerät (15) der Personentransportanlage (1) an einen entfernt angeordneten Computer (35) zu senden und/oder Daten von einem entfernt angeordneten Computer (35) für einen Empfang durch das Steuergerät (15) der Personentransportanlage (1) bereitzustellen.

12. Personentransportanlage nach einem der Ansprüche 2 bis 11,
wobei das Steuergerät (15) die Steuersignale unter Berücksichtigung von Informationen über eine Geschwindigkeit, mit der die Personenfördereinrichtung (13) betrieben wird, und/oder eine Anzahl von mit der Personenfördereinrichtung (13) beförderten Personen generiert.

13. Personentransportanlage nach einem der Ansprüche 2 bis 12,
wobei die Desinfektionseinrichtung (23) ferner eine Ausgabeeinrichtung (39) aufweist, welche dazu eingerichtet ist, ein für einen Menschen wahrnehmbares Signal auszugeben, welches einen aktuellen Zustand der Desinfektionseinrichtung (23) angibt.

14. Verfahren zum Betreiben einer Personentransportanlage (1) gemäß einem der vorangehenden Ansprüche 2 bis 13, wobei das Verfahren aufweist:
- Übermitteln von Statussignalen, welche Informationen über einen aktuellen Betrieb, einen aktuellen Zustand und/oder eine aktuelle Konfiguration der Desinfektionseinrichtung (23) wiedergeben, von der Steuerung (25) an das Steuergerät (15), und
- Steuern eines Betriebs der Desinfektionseinrichtung (23) unter Berücksichtigung empfangener Steuersignale, welche von dem Steuergerät (15) unter Berücksichtigung empfangener Statussignale an die Steuerung (25) übermittelt werden,
wobei ferner die Statussignale optional von dem Steuergerät (15) an einen entfernt angeordneten Computer (35) gesendet und dort verarbeitet werden und/oder wobei ferner Steuersignale optional von dem entfernt angeordneten Computer (35) an das Steuergerät (15) und von diesem weiter an die Steuerung (25) übermittelt werden, um den Betrieb der Desinfektionseinrichtung (23) zu steuern.

15. Verfahren nach Anspruch 14,
wobei in dem entfernt angeordneten Computer (35) ein digitaler Zwilling der Personentransportanlage (1) gespeichert ist und wobei Eigenschaften der Personentransportanlage (1) basierend auf dem digitalen Zwilling der Personentransportanlage (1) unter Berücksichtigung der von der Steuerung (25) der Desinfektionseinrichtung (23) übermittelten Statussignale ermittelt werden.

## Claims

1. A disinfection device (23) for disinfecting a surface (41) on a passenger conveying device (13), which passenger conveying device (13) is part of a passenger transport system (1) and is used for conveying passengers within a building structure (5); wherein the disinfection device (23) comprises a controller (25) for controlling an operation of the disinfection device (23);
wherein the controller (25) of the disinfection device (23) is adapted to be connected to a control unit (15) of the passenger transport system (1) via a bidirectional communication system (33), and
wherein the controller (25) of the disinfection device (23) is adapted to send status signals, which reflect information about a current operation, a current state and/or a current configuration of the disinfection device (23), to the control unit (15) and to receive control signals generated by the control unit (15) of the passenger transport system (1) taking into account received status signals, wherein the controller (25) controls the operation of the disinfection device (23) taking into account received control signals, **characterized in that** the controller (25) of the disinfection device (23) is adapted to control a disinfection intensity with which the disinfection device (23) disinfects the surface (41) to be disinfected, depending on control signals which are transmitted to the controller (25) by the control unit (15) of the passenger transport system (1) and which indicate an operating parameter of the disinfection device (23) correlating with a disinfection intensity currently to be effected.

2. A passenger transport system (1), comprising:
- a passenger conveying device (13) for conveying passengers within a building structure (5);
- a control unit (15) for controlling an operation of the passenger conveying device (13); and
- a disinfecting device (23) according to claim 1 for disinfecting a surface (41) to be disinfected on the passenger conveying device (13);
wherein the disinfection device (23) comprises a controller (25) for controlling an operation of the disinfection device (23);
wherein the controller (25) of the disinfection device (23) is connected to the control unit (15) of the passenger transport system (1) via a bidirectional communication system (33), and
wherein the control unit (15) of the passenger transport system (1) is adapted in such a manner that status signals, which reflect information about a current operation, a current state and/or a current configuration of the disinfection device (23), are received from the controller (25), and the control unit (15) generates control signals taking into account received status signals and transmits them to the controller (25), wherein the controller (25) controls the operation of the disinfection device (23) taking into account received control signals.

3. The passenger transport system according to claim 2,
wherein the control unit (15) generates control signals which indicate a setpoint value for the operating parameter of the disinfection device (23) correlating with a disinfection intensity currently to be effected, taking into account the status signals received from the controller (25).

4. The passenger transport system according to any one of claim 2 or 3,
wherein the disinfection device (23) is adapted to be able, in a new state, to effect as maximum disinfection intensity a disinfection intensity which is greater than a minimum disinfection intensity necessary for sufficient disinfection of the surface (41) to be disinfected, and
wherein the control unit (15) of the passenger transport system (1) and/or the controller (25) of the disinfection device (23) are adapted to operate the disinfection device (23) in normal operation with a disinfection intensity above the minimum disinfection intensity and to successively increase an operating parameter of the disinfection device (23) correlating with a currently effected disinfection intensity.

5. The passenger transport system according to any one of claims 2 to 4,
wherein the control unit (15) of the passenger transport system (1) and/or the controller (25) of the disinfection device (23) are adapted to increase the operating parameter of the disinfection device (23) correlating with a currently effected disinfection intensity depending on an operating time since start-up of the disinfection device (23).

6. The passenger transport system according to any one of claims 2 to 5,
wherein the disinfection device (23) further comprises a sensor (29) for detecting a measured variable which correlates with the disinfection intensity, and
wherein the control unit (15) of the passenger transport system (1) and/or the controller (25) of the disinfection device (23) are adapted to increase the operating parameter of the disinfection device (23) correlating with a currently effected disinfection intensity depending on the measured variable detected by the sensor (29).

7. The passenger transport system according to claim 6,
wherein the control unit (15) of the passenger transport system (1) and/or the controller (25) of the disinfection device (23) are adapted to regulate the operating parameter of the disinfection device (23) correlating with a currently effected disinfection intensity depending on the measured variable detected by the sensor (29).

8. The passenger transport system according to any one of claims 2 to 7,
wherein the operating parameter of the disinfection device (23) correlating with a disinfection intensity currently to be effected is monitored and, if the operating parameter exceeds a predetermined threshold value, a warning signal is output which indicates an approaching end of a service life of the disinfection device (23).

9. The passenger transport system according to any one of claims 2 to 8,
wherein the disinfection device (23) further comprises a sensor (31) for detecting a temperature inside the disinfection device (23) and/or for detecting a temperature in an environment adjacent to the disinfection device (23), and
wherein the control unit (15) of the passenger transport system (1) and/or the controller (25) of the disinfection device (23) are adapted to control the operation of the disinfection device (23) depending on the temperature detected by the sensor (31).

10. The passenger transport system according to claim 6 or 9,
wherein the controller (25) of the disinfection device (23) is adapted to send the measured variable determined by the sensor (29, 31) and/or a data signal correlating with the measured variable determined by the sensor (29, 31) to the control unit (15) of the passenger transport system (1).

11. The passenger transport system according to any one of claims 2 to 10,
wherein the passenger transport system (1) further comprises an interface device (37) adapted to send data from the control unit (15) of the passenger transport system (1) to a remotely arranged computer (35) and/or to provide data from a remotely arranged computer (35) for reception by the control unit (15) of the passenger transport system (1).

12. The passenger transport system according to any one of claims 2 to 11,
wherein the control unit (15) generates the control signals taking into account information about a speed at which the passenger conveying device (13) is operated and/or a number of passengers conveyed by the passenger conveying device (13).

13. The passenger transport system according to any one of claims 2 to 12, wherein the disinfection device (23) further comprises an output device (39), which is adapted to output a signal which can be perceived by a human being and which indicates a current state of the disinfection device (23).

14. A method for operating a passenger transport system (1) according to any of the preceding claims 2 to 13, the method comprising:
- transmitting status signals reflecting information about a current operation, a current state and/or a current configuration of the disinfection device (23) from the controller (25) to the control unit (15), and
- controlling an operation of the disinfection device (23) taking into account received control signals which are transmitted from the control unit (15) to the controller (25) taking into account received status signals,
wherein, furthermore, the status signals are optionally sent from the control unit (15) to a remotely arranged computer (35) and processed there and/or wherein, furthermore, control signals are optionally transmitted from the remotely arranged computer (35) to the control unit (15) and from the control unit further to the controller (25) to control the operation of the disinfection device (23).

15. The method according to claim 14,
wherein a digital twin of the passenger transport system (1) is stored in the remotely arranged computer (35) and wherein properties of the passenger transport system (1) are determined based on the digital twin of the passenger transport system (1) taking into account the status signals transmitted by the controller (25) of the disinfection device (23).

## Revendications

1. Dispositif de désinfection (23) permettant la désinfection d'une surface (41) sur un dispositif de transport de personnes (13), lequel dispositif de transport de personnes (13) fait partie d'une installation de transport de personnes (1) et sert au transport de personnes à l'intérieur d'un bâtiment (5) ;
dans lequel le dispositif de désinfection (23) présente une commande (25) pour la commande d'un fonctionnement du dispositif de désinfection (23) ;
dans lequel la commande (25) du dispositif de désinfection (23) est destinée à être reliée à un appareil de commande (15) de l'installation de transport de personnes (1) par l'intermédiaire d'un système de communication bidirectionnel (33), et
dans lequel la commande (25) du dispositif de désinfection (23) est destinée à envoyer à l'appareil de commande (15) des signaux d'état qui reproduisent des informations sur un fonctionnement actuel, un état actuel et/ou une configuration actuelle du dispositif de désinfection (23), et pour recevoir des signaux de commande générés par l'appareil de commande (15) de l'installation de transport de personnes (1) en tenant compte des signaux d'état reçus, dans lequel la commande (25) commande le fonctionnement du dispositif de désinfection (23) en tenant compte des signaux de commande reçus, **caractérisé en ce que** la commande (25) du dispositif de désinfection (23) est destinée à commander une intensité de désinfection avec laquelle le dispositif de désinfection (23) désinfecte la surface (41) à désinfecter, en fonction de signaux de commande qui sont transmis à la commande (25) par l'appareil de commande (15) de l'installation de transport de personnes (1) et qui indiquent un paramètre de fonctionnement du dispositif de désinfection (23) en corrélation avec une intensité de désinfection actuelle devant être provoquée.

2. Installation de transport de personnes (1), présentant :
- un dispositif de transport de personnes (13) pour le transport de personnes à l'intérieur d'un bâtiment (5) ;
- un appareil de commande (15) pour la commande d'un fonctionnement du dispositif de transport de personnes (13) ; et
- un dispositif de désinfection (23) selon la revendication 1 pour la désinfection d'une surface (41) à désinfecter sur le dispositif de transport de personnes (13) ;
dans laquelle le dispositif de désinfection (23) présente une commande (25) pour la commande d'un fonctionnement du dispositif de désinfection (23) ;
dans laquelle la commande (25) du dispositif de désinfection (23) est reliée à un appareil de commande (15) de l'installation de transport de personnes (1) par l'intermédiaire d'un système de communication bidirectionnel (33) et
dans laquelle l'appareil de commande (15) de l'installation de transport de personnes (1) est destinée à recevoir de la commande (25) des signaux d'état qui reproduisent des informations sur un fonctionnement actuel, un état actuel et/ou une configuration actuelle du dispositif de désinfection (23), et l'appareil de commande (15) génère des signaux de commande en tenant compte des signaux d'état reçus et les transmet à la commande (25), dans laquelle la commande (25) commande le fonctionnement du dispositif de désinfection (23) en tenant compte des signaux de commande reçus.

3. Installation de transport de personnes selon la revendication 2,
dans laquelle l'appareil de commande (15) génère des signaux de commande qui indiquent une valeur de consigne pour le paramètre de fonctionnement du dispositif de désinfection (23) en corrélation avec une intensité de désinfection devant être provoquée actuelle, en tenant compte des signaux d'état reçus par la commande (25).

4. Installation de transport de personnes selon l'une des revendications 2 ou 3,
dans laquelle le dispositif de désinfection (23) est destiné à pouvoir provoquer une intensité de désinfection dans un état neuf en tant qu'intensité de désinfection maximale,
qui est supérieure à une intensité de désinfection minimale nécessaire pour une désinfection suffisante de la surface (41) à désinfecter, et
dans laquelle l'appareil de commande (15) de l'installation de transport de personnes (1) et/ou la commande (25) du dispositif de désinfection (23) sont destinés à faire fonctionner le dispositif de désinfection (23) en fonctionnement normal avec une intensité de désinfection au-dessus de l'intensité de désinfection minimale et pour augmenter successivement un paramètre de fonctionnement du dispositif de désinfection (23) en corrélation avec une intensité de désinfection provoquée actuelle.

5. Installation de transport de personnes selon l'une des revendications 2 à 4, dans laquelle l'appareil de commande (15) de l'installation de transport de personnes (1) et/ou la commande (25) du dispositif de désinfection (23) sont destinés à augmenter le paramètre de fonctionnement du dispositif de désinfection (23) en corrélation avec une intensité de désinfection provoquée actuelle, en fonction d'une durée de fonctionnement depuis la mise en service du dispositif de désinfection (23).

6. Installation de transport de personnes selon l'une des revendications 2 à 5,
dans laquelle le dispositif de désinfection (23) présente en outre un capteur (29) pour la détection d'une grandeur de mesure qui est en corrélation avec l'intensité de désinfection, et
dans laquelle l'appareil de commande (15) de l'installation de transport de personnes (1) et/ou la commande (25) du dispositif de désinfection (23) sont destinés à augmenter le paramètre de fonctionnement du dispositif de désinfection (23) en corrélation avec une intensité de désinfection provoquée actuelle, en fonction de la grandeur de mesure détectée par le capteur (29).

7. Installation de transport de personnes selon la revendication 6,
dans laquelle l'appareil de commande (15) de l'installation de transport de personnes (1) et/ou la commande (25) du dispositif de désinfection (23) sont destinés à réguler le paramètre de fonctionnement du dispositif de désinfection (23) en corrélation avec une intensité de désinfection provoquée actuelle, en fonction de la grandeur de mesure détectée par le capteur (29).

8. Installation de transport de personnes selon l'une des revendications 2 à 7,
dans laquelle le paramètre de fonctionnement du dispositif de désinfection (23) en corrélation avec une intensité de désinfection à provoquer actuelle est surveillé et, en cas de dépassement du paramètre de fonctionnement au-delà d'une valeur seuil prédéterminée, un signal d'avertissement est émis, lequel indique une fin imminente d'une durée de vie du dispositif de désinfection (23).

9. Installation de transport de personnes selon l'une des revendications 2 à 8,
dans laquelle le dispositif de désinfection (23) présente en outre un capteur (31) pour la détection d'une température à l'intérieur du dispositif de désinfection (23) et/ou pour la détection d'une température dans un environnement adjacent au dispositif de désinfection (23), et
dans laquelle l'appareil de commande (15) de l'installation de transport de personnes (1) et/ou la commande (25) du dispositif de désinfection (23) sont destinés à commander le fonctionnement du dispositif de désinfection (23) en fonction de la température déterminée par le capteur (31).

10. Installation de transport de personnes selon la revendication 6 ou 9,
dans laquelle la commande (25) du dispositif de désinfection (23) est destinée à envoyer à l'appareil de commande (15) de l'installation de transport de personnes (1) la grandeur de mesure déterminée par le capteur (29, 31) et/ou un signal de données en corrélation avec la grandeur de mesure déterminée par le capteur (29, 31).

11. Installation de transport de personnes selon l'une des revendications 2 à 10,
dans laquelle l'installation de transport de personnes (1) présente en outre un dispositif d'interface (37) qui est destiné à envoyer des données de l'appareil de commande (15) de l'installation de transport de personnes (1) à un ordinateur (35) disposé à distance et/ou pour mettre à disposition des données d'un ordinateur (35) disposé à distance pour une réception par l'appareil de commande (15) de l'installation de transport de personnes (1).

12. Installation de transport de personnes selon l'une des revendications 2 à 11,
dans laquelle l'appareil de commande (15) génère les signaux de commande en tenant compte d'informations sur une vitesse à laquelle le dispositif de transport de personnes (13) fonctionne,
et/ou d'un nombre de personnes transportées par le dispositif de transport de personnes (13).

13. Installation de transport de personnes selon l'une des revendications 2 à 12,
dans laquelle le dispositif de désinfection (23) présente en outre un dispositif de sortie (39) qui est destiné à émettre un signal perceptible par un être humain, qui indique un état actuel du dispositif de désinfection (23).

14. Procédé permettant de faire fonctionner une installation de transport de personnes (1) selon l'une des revendications 2 à 13, dans lequel le procédé présente :
- la transmission de signaux d'état, qui reproduisent des informations sur un fonctionnement actuel, un état actuel et/ou une configuration actuelle du dispositif de désinfection (23), de la commande (25) à l'appareil de commande (15), et
- la commande d'un fonctionnement du dispositif de désinfection (23) en tenant compte de signaux de commande reçus, qui sont transmis par l'appareil de commande (15) à la commande (25) en tenant compte de signaux d'état reçus,
dans lequel, en outre, les signaux d'état sont envoyés en option par l'appareil de commande (15) à un ordinateur (35) disposé à distance et y sont traités et/ou dans lequel, en outre, des signaux de commande sont transmis en option par l'ordinateur (35) disposé à distance à l'appareil de commande (15) et, de là, à la commande (25), afin de commander le fonctionnement du dispositif de désinfection (23).

15. Procédé selon la revendication 14,
dans lequel un jumeau numérique de l'installation de transport de personnes (1) est mémorisé dans l'ordinateur (35) disposé à distance et dans lequel des propriétés de l'installation de transport de personnes (1) sont déterminées sur la base du jumeau numérique de l'installation de transport de personnes (1) en tenant compte des signaux d'état transmis par la commande (25) du dispositif de désinfection (23).
